# FASCICULE DE BREVET EUROPEEN

(11) **EP 0 639 071 B1**
(45) Date de publication et mention de la délivrance du brevet: **12.08.1998**
(21) Numéro de dépôt: 94909151.6
(22) Date de dépôt: 04.03.1994
(51) Int. Cl.: A61K 9/10, A61K 47/14, A61K 47/06

(54) **EMULSIONS VACCINALES FLUIDES EAU-DANS-L'HUILE CONTENANT UNE HUILE METABOLISABLE**
Flüssige Wasser in Oel Impfemulsion enthaltend ein metabolierbares Oel
WATER-IN-OIL FLUID VACCINAL EMULSIONS CONTAINING A METABOLIZABLE OIL

(30) Priorité: 08.03.1993 FR 9302661; 07.12.1993 FR 9314651
(43) Date de publication de la demande: 22.02.1995
(73) Titulaire: MERIAL, 69002 Lyon (FR)
(72) Inventeur: RIVIERE, Michel, Emile, Albert, F-69130 Ecully (FR); ROULET, Claude, F-69200 Vénissieux (FR)
(74) Mandataire: Bernasconi, Jean
(86) Numéro de dépôt international: FR9400242
(87) Numéro de publication internationale: WO9420071

(56) Documents cités:
- EP-A- 0 174 377
- WO-A-91/00107
- BE-A- 648 053
- BE-A- 678 818
- DE-A- 1 492 255
- FR-A- 2 466 247
- FR-M- 4 690
- GB-A- 2 189 457
- US-A- 3 966 632
- DATABASE WPI Section Ch, Week 9344, Derwent Publications Ltd., London, GB; Class A06, AN 93-348363 & JP,A,5 255 112 (KANEBO LTD) 5 Octobre 1993
- PATENT ABSTRACTS OF JAPAN vol. 11, no. 159 (C-423)22 Mai 1987 & JP,A,61 289 026 (NIPPON OIL & FATS CO LTD) 19 Décembre 1986
- 4-Toxicology, vol. 111, 1989, abstract 128452b
- "Intestinal Absorption and metabolism of hydrocarbons", J.A.Barrowman et al., Prog. Lipid Res., vol. 28, pp 189-203, 1989

## Description

La présente invention concerne principalement des émulsions injectables eau-dans-l'huile entièrement métabolisables, destinées à stimuler la réponse immunitaire induite par des principes actifs, qui peuvent être des virus, des bactéries, des parasites ou des fractions de ceux-ci, des toxines, des polysaccharides, mais aussi des protéines recombinantes, ou bien des petites molécules antigéniques, par exemple des peptides, couplées sur des molécules porteuses.

### Rappel des connaissances

La vaccination, en stimulant le système de défense immunitaire, est un moyen de lutte préventive contre les agents infectieux. Les vaccins utilisent actuellement comme antigène soit des microorganismes vivants dont le pouvoir pathogène a été atténué, soit des microorganismes tués, voire même des fractions purifiées à partir de ces microorganismes. Très souvent les vaccins inactivés ou de sous-unités contiennent une substance adjuvante qui est destinée à accroître la réponse immunitaire. La fonction de l'adjuvant est d'augmenter d'une part le niveau de la réponse immunitaire humorale et cellulaire, d'autre part la durée de cette réponse. Par vole de conséquence, l'adjuvant permet de réduire le nombre d'injections et la dose d'antigène incluse dans le vaccin, maintenant ainsi la vaccination à un coût acceptable. En dehors de l'effet recherché sur la réponse immunitaire, les substances adjuvantes peuvent induire une toxicité locale ou générale : oedème inflammatoire, abcès, fibrose au point d'injection, douleur, fièvre, stimulation d'un état d'hypersensibilité. Un adjuvant doit être efficace mais également être acceptable au plan toxicité.

Parmi les nombreuses substances adjuvantes de l'immunité, on peut citer les gels dérivés de l'aluminium (hydroxyde, phosphate), qui sont seuls utilisés en médecine humaine, des saponines, qui sont des hétérosides complexes et qui sont employées dans le domaine vétérinaire, et aussi les émulsions qui semblent être l'un des excipients les plus actifs. En particulier, les émulsions contenant des mycobactéries dans l'huile minérale et comme tensioactif l'Arlacel A®, qui est défini comme un monooléate de dianhydromannitol, sont largement utilisées sous le nom d'adjuvant de Freund complet, pour l'immunisation d'animaux de laboratoire. Néanmoins, l'utilisation des mycobactéries tuées présente deux défauts majeurs : les réactions locales au point d'injection sont très importantes et les animaux sont sensibilisés à la tuberculine ; cela conduit à rejeter cet adjuvant en médecine vétérinaire. Le même adjuvant sans mycobactéries, appelé adjuvant de Freund incomplet, est moins mal toléré et est encore largement utilisé dans les laboratoires pour la production de sérums hyperimmuns. Toutefois, les émulsions préparées avec les adjuvants de Freund sont très visqueuses et peu stables, ce qui empêche leur développement industriel. La recherche des substances responsables de l'effet adjuvant des mycobactéries a conduit à la caractérisation des peptidoglycanes (MDP) et des glycolipides (TMD), puis à leur emploi (ou celui de leurs dérivés) dans les émulsions à la place des mycobactéries, de manière à réduire la toxicité de ces émulsions (WOODWARD L.F., 1990, Surface Chemistry and Classification of vaccine adjuvants and vehicles, in Bacterial Vaccines pp. 281-306, Alan R. Liss, New York).

L'emploi d'émulsions dans les vaccins est resté longtemps anecdotique ; les essais qui ont été effectués, chez l'homme en particulier, avec d'une part un vaccin grippe en adjuvant de type Freund incomplet, d'autre part une émulsion renfermant de l'huile d'arachide, de l'Arlacel A® et du stéarate d'aluminium connue sous le nom de "adjuvant 65" ont finalement conduit à proscrire de telles formules de la médecine humaine.

Il a fallu attendre les travaux de Cessi et Nardelli (Develop. Biol. Standard., 1973, 25, 325-328) pour voir se développer sur un plan industriel des vaccins en émulsion eau-dans-l'huile. Ces vaccins étaient destinés à protéger les poules pondeuses contre la maladie de Newcastle, due à un paramyxovirus, pendant toute la durée de la ponte, ce dont étaient incapables les vaccins classiques pour lesquels l'immunité plus faible se traduisait par une chute de la ponte lors d'un passage de virus à travers l'élevage. Les vaccins de ce type se sont rapidement développés dans le monde de l'aviculture, en Europe au moins, pour de nombreuses maladies aviaires (par exemple bronchite infectieuse aviaire, maladie de Gumboro). Sont également apparus sur le marché des vaccins à base d'émulsion, plus particulièrement de type huile-dans-l'eau, pour la prévention de plusieurs maladies des mammifères, d'origine bactérienne (brucellose des ruminants, entérotoxémie des ovins, colibacillose) ou virale (fièvre aphteuse du porc, grippe porcine, maladie d'Aujeszky...). Toutefois, s'ils présentent généralement une activité immunogène très supérieure à celle des vaccins non huileux, ces vaccins peuvent provoquer des réactions locales au site d'injection du vaccin. Ces réactions, dont l'intensité dépend de la formulation employée (eau-dans-l'huile ou huile-dans- l'eau) et aussi de la nature des antigènes, sont classiquement attribuées à la présence de l'huile minérale. En effet, celle-ci ne semble pas être métabolisée par l'organisme et reste en partie à proximité des sites d'injection. Il était donc souhaitable de remplacer l'huile minérale par une huile métabolisable.

Par huile métabolisable, il faut entendre des hydrocarbures naturels présents dans la nature, tels que le squalène, le squalane et le pristane, les huiles végétales appartenant à la catégorie des triglycérides, des triglycérides d'hémisynthèse, comme la trioléine et les triglycérides à chaîne moyenne (C8/C10), les esters d'acides gras, et plus particulièrement les oléates d'oléyle, le dioléate de propylèneglycol, les diesters des acides caprique/caprylique et du propylèneglycol.

A ce stade, une difficulté est que les émulsions doivent être injectables, donc assez fluides ; cela amène à privilégier les triglycérides à chaîne moyenne et certains esters, plutôt que les huiles végétales, dont la viscosité à 25°C est déjà appréciable (50 à 70 mPa.s).

Mais la difficulté majeure est d'obtenir des émulsions eau-dans-l'huile stables avec ces huiles, et les huiles végétales en particulier. Pour ce faire, l'utilisation des monoglycérides est largement répandue dans l'industrie alimentaire, mais en général les émulsions recherchées et obtenues sont épaisses, ce qui favorise leur stabilité.

Plusieurs formules d'émulsion de type eau-dans-l'huile à base d'huiles métabolisables ont déjà été publiées : huile d'arachide et lécithine (BRUGH M. et al., Am. J. Vet. Res. 1983, 44, 72-75), huile enrichie en diglycérides et phospholipides modifiés (demande de brevet EP-A-0 417 562), triglycérides ou esters gras associés à des hydrocarbures et à des tensioactifs (brevet français n° 2 649 013). Aucune de ces formules d'émulsions ne correspond à la présente invention.

La demande de brevet internationale WO 91/00107 décrit la préparation d'émulsions à partir d'un mélange d'huile métabolisable et d'huile non métabolisable (de 2 à 95 %) et de tensioactifs émulgateurs obtenus par condensation d'un acide gras liquide à 20°C sur un sucre, tel que mannitol, glucose ou saccharose, ou sur du glycérol. Les tensioactifs choisis ne permettent pas de réaliser une émulsion sur la base uniquement d'huile métabolisable.

D'autre part, dans la demande de brevet EP-A-0 174 377, des polyricinoléates de polyglycérol ont été utilisés comme émulsifiant dans une application toute particulière pour la préparation de doubles émulsions eau-huile-eau destinées à posséder une grande résistance à la chaleur, à la congélation et au stockage, dans le domaine des produits cosmétiques et médicaux à usage externe et des médicaments classiques, par exemple l'insuline. Le problème technique que cette demande antérieure visait à résoudre était notamment celui d'éviter une dénaturation de l'émulsion lors des traitements finaux de stérilisation ou analogue, ce qui n'a pas de point commun avec la recherche d'émulsions adjuvantes selon l'invention.

Le document DE-A-1 492 255 décrit un vaccin.

Nous avons découvert, de façon surprenante, que l'utilisation de ricinoléate de polyglycérol ou de polyricinoléate de polyglycérol, seul ou en association avec d'autres tensioactifs tels que les monoglycérides, les huiles de ricin polyoxyéthylénées, hydrogénées ou non, ou bien les esters de sorbitan, conduit à des émulsions eau-dans-l'huile très stables et fluides, cela avec comme huile tant un triglycéride, qui peut être une huile végétale ou des triglycérides dits à chaîne moyenne (tricaprylate/tricaprate de glycérol), que des esters du propylèneglycol (dioléate ou bien dicaprylate/dicaprate), ou encore des esters de type oléate d'oléyle ou de décyle. Ces émulsions eau-dans-l'huile ont un pouvoir adjuvant. Ces formules peuvent être valablement étendues aux hydrocarbures naturels, par exemple squalène et squalane.

Nous avons découvert d'autre part que certains tensioactifs appartenant à la série des huiles de ricin polyoxyéthylénées, hydrogénées ou non, conduisent, avec certaines huiles, et plus particulièrement avec certains esters de l'acide oléique (notamment avec l'alcool oléique ou le propylèneglycol), à des émulsions eau-dans-l'huile qui sont très stables, fluides à 4°C et ont un pouvoir adjuvant.

Par grande stabilité au sens de la présente invention, on entend une stabilité qui se traduit par un aspect macroscopiquement homogène, par exemple à l'oeil nu, pendant une durée compatible avec la durée de stockage possible du produit ou vaccin contenant l'émulsion, durée de préférence de plusieurs mois et pouvant être supérieure à une année.

Il est ainsi devenu possible de réaliser des émulsions à base d'huiles métabolisables qui sont extrêmement stables et ont un fort pouvoir adjuvant, tout en présentant une excellente tolérance, et qui sont fluides à basse température, notamment à 4°C.

La présente invention a donc pour objet une émulsion injectable eau-dans-l'huile réunissant pour la première fois l'utilisation exclusive d'huile métabolisable, ce qui permet d'obtenir une très bonne tolérance, et l'obtention d'une émulsion stable et ayant la fluidité requise pour en permettre son administration par injection, ainsi qu'un caractère adjuvant de l'immunité. Par rapport aux émulsions de l'art antérieur, du type comportant une phase huileuse avec agent émulsionnant ou tensioactif émulgateur, l'émulsion selon l'invention se caractérise par le fait que l'huile de la phase huileuse est une huile métabolisable ou un mélange d'huiles métabolisables et l'agent émulsionnant, ou mélange d'agents émulsionnants, est adapté à l'obtention, avec l'huile métabolisable, d'une émulsion stable et à viscosité relativement faible (moins de 400 mPa.s à 25°C) et restant avantageusement fluide à 4°C.

De préférence, l'agent émulsionnant ou tensioactif émulgateur est à base d'ester de l'acide ricinoléique et/ou d'ester de polyglycérol. Comme on le verra plus loin, certains tensioactifs selon l'invention sont à la fois des esters de polyglycérol et des esters de l'acide ricinoléique. Ce sont notamment les ricinoléates et polyricinoléates de polyglycérol. On comprend que l'invention porte sur la découverte que ces tensioactifs permettent d'utiliser uniquement des huiles métabolisables et que ces tensioactifs pourront être avantageusement ou même préférentiellement employés en présence d'autres tensioactifs classiques.

La phase huileuse métabolisable peut être constituée d'hydrocarbures naturels, par exemple le squalane (hexaméthyl-2, 6, 10, 15, 19, 23 tétracosane), le squalène (hexaméthyl-2, 6, 10, 15, 19, 23 tétracosahexaène-2, 6, 10, 14, 18, 22), le pristane (tétraméthyl-2, 6, 10, 14 pentadécane).

La phase huileuse métabolisable peut être avantageusement constituée d'huile végétale, de préférence de la série des triacylglycérols, notamment huile de tournesol, de soja, de maïs, d'amande douce.

La phase huileuse métabolisable peut encore être avantageusement et même préférentiellement constituée d'esters d'acides gras naturels et d'alcools.

Les alcools peuvent être seuls ou en mélange :
- des polyols tels que le pentaérythritol, mais plus particulièrement le glycérol et/ou le propylèneglycol (encore appelé propanediol-1, 2) ;
- des monoalcools aliphatiques à chaîne linéaire, tels que l'isopropanol et les alcools primaires, comme l'éthanol, le butanol, l'octanol-1, le décanol-1, et plus particulièrement l'alcool oléique (ou octadécène-9 ol-1) ;
- des monoalcools aliphatiques à chaîne ramifiée, tels que l'éthyl-2 hexanol-1 et l'alcool dit isocétylique.

Les acides gras majoritaires sont de préférence des acides gras naturels de C6 à C24, de préférence l'acide oléique et/ou linoléique, l'acide caprique et/ou caprylique.

Les acides gras majoritaires peuvent aussi être des diacides tels que l'acide succinique ou l'acide adipique, ou bien des acides gras ramifiés, et plus particulièrement l'acide isostéarique.

Parmi les différents esters, on préfère notamment l'oléate d'oléyle, les diesters de propylèneglycol, plus particulièrement le dioléate ou le dicaprate dicaprylate de propylèneglycol.

Dans un premier mode de mise en oeuvre préféré, les tensioactifs émulgateurs contiennent en totalité ou en partie des esters de polyglycérol, en proportion finale de 0,4 à 20 % p/v, de préférence entre 1 et 10 % et plus particulièrement entre 3 et 5 % p/v.

Les esters de polyglycérol peuvent être en totalité ou en partie des esters de polyglycérol et d'acides gras naturels, tels que l'acide oléique, l'acide stéarique, l'acide ricinoléique, ou bien des acides gras ramifiés tels que l'acide isostéarique.

Les esters de polyglycérol peuvent être en totalité ou en partie des ricinoléates (hydroxy-12 octadécène-9 oate) de polyglycérol.

Par exemple, les esters de polyglycérol sont en totalité ou en partie des polyricinoléates de polyglycérol.

De façon avantageuse, le polyglycérol possède de 2 à 12 résidus glycérol, et plus particulièrement de 2 à 5 résidus.

L'ester de polyglycérol peut être associé à d'autres tensioactifs, en particulier l'oléate de sorbitan, les ricins polyoxyéthylénés, hydrogénés ou non (voir plus loin les huiles de ricin selon l'invention), et/ou des tensioactifs naturels. D'une manière particulière, l'ester de polyglycérol pourra être associé en faible quantité à d'autres tensioactifs, notamment huiles de ricin selon l'invention, en particulier à une concentration inférieure à 1 % p/v, notamment de l'ordre de 0,4 à 1 % p/v.

Les tensioactifs naturels sont, par exemple, la lécithine ou bien les glycérides (mono + di), plus particulièrement les glycérides d'acides gras insaturés (oléique et/ou linoléique majoritaires) et d'acides gras à chaîne moyenne (caprylique et/ou caprique).

Dans un autre mode de réalisation préféré, les tensioactifs émulgateurs comprennent, en totalité ou en partie, des huiles de ricin polyoxyéthylénées, hydrogénées ou non. De préférence, cette ou ces huiles de ricin polyoxyéthylénées sont en proportion finale de 0,5 à 25 % p/v, de préférence entre 2 et 10 % et plus particulièrement entre 4 et 8 %.

De façon avantageuse, les huiles de ricin polyoxyéthylénées, hydrogénées ou non, ont un degré d'éthoxylation inférieur à 18 moles d'oxyde d'éthylène par mole.

Les huiles de ricin polyoxyéthylénées, hydrogénées ou non, peuvent être associées entre elles et/ou à d'autres tensioactifs appartenant à des séries chimiques différentes, en particulier les dérivés du sorbitan, les alcools ou les acides gras polyoxyéthylénés, les glycérides, les lécithines. Comme on l'a vu plus haut, les huiles de ricin peuvent aussi être associées aux esters du polyglycérol selon l'invention et d'une manière générale à tous les tensioactifs indiqués dans le premier mode de réalisation.

Avantageusement, la phase aqueuse de l'émulsion est en proportion telle que la viscosité de l'émulsion soit acceptable, pour que ladite émulsion puisse être facilement injectée, de préférence entre 5 et 50 % et plus particulièrement entre 12 et 25 % v/v.

L'émulsion peut contenir des substances modulatrices de l'immunité.

L'invention concerne également les vaccins formés d'une émulsion selon l'invention et caractérisés en ce que la phase aqueuse de l'émulsion selon l'invention contient, seuls ou en association, des antigènes entiers d'origine virale, bactérienne, parasitaire, ou bien des fractions desdits antigènes, ou des protéines obtenues par recombinaison génétique.

Elle concerne également les médicaments immunologiques caractérisés en ce que la phase aqueuse de l'émulsion selon l'invention contient une protéine étrangère, ou une protéine modifiée, ou un haptène conjugué à une molécule porteuse.

L'invention concerne bien entendu aussi les procédés de préparation des émulsions, vaccins et médicaments immunologiques décrits ci-dessus, consistant dans la mise en présence adéquate des constituants et l'émulsification.

L'invention a encore pour objet l'utilisation des esters de polyglycérol et des esters de l'acide ricinoléique selon l'invention pour la réalisation d'émulsions eau-dans-l'huile à activité adjuvante, stables et à viscosité relativement faible, et comprenant une phase huileuse faite d'huile métabolisable majoritaire et d'huile non métabolisable minoritaire.

Nous donnerons ci-après, à titre d'illustration de la présente invention, quelques exemples de formules et leurs propriétés. Il va de soi, pour les personnes expertes en l'art, que les proportions de phases et les concentrations de tensioactifs qui ont été utilisées dans les exemples ci-après peuvent s'étendre dans un domaine de variation beaucoup plus large. De même, ces personnes sont à même de préparer et essayer de manière usuel le toute association d'un tensioactif classique et d'un tensioactif selon l'invention.

### Exemple n° 1

Cet exemple est destiné à illustrer l'efficacité des polyricinoléates de polyglycérol comme tensioactifs.

Dans 800 ml d'oléate d'oléyle (Cétiol, Henkel) contenant 5 % p/v de polyricinoléate de polyglycérol (Radiamuls 2253, Oléofina), 200 ml de tampon isotonique sont émulsifiés à l'aide d'un émulsionneur à turbine (Silverson).

L'émulsion obtenue est très stable (début de décantation mais pas de trace de phase aqueuse continue après 1 an à 4°C, de même qu'à température ambiante) et fluide (170 mPa.s à 4°C, 60 mPa.s à 25°C).

L'utilisation d'huile d'amande douce à la place de l'oléate d'oléyle conduit aussi à une émulsion eau-dans-l'huile stable à 4°C ainsi qu'à température ambiante, et relativement fluide (360 mPa.s à 5°C, 140 mPa.s à 25°C).

L'huile de soja conduit elle aussi à une émulsion stable, tout comme les triglycérides à chaînes moyennes (C8-C10) ainsi que les diesters du propylèneglycol (oléique ou bien caprylique/caprique).

### Exemple n° 2

Cet exemple est destiné à illustrer les propriétés tensioactives de l'Arlacel 1689 (ICI) qui contient du ricinoléate de polyglycérol-3 et de l'oléate de sorbitan. 35 ml de phase aqueuse constituée d'un tampon isotonique contenant l'antigène sont émulsifiés à l'aide d'un émulsionneur à turbine (Silverson) dans 140 ml de phase huileuse formée d'une solution d'Arlacel 1689 (ICI) à 5 % (p/v) dans l'huile ; l'huile étant indifféremment soit de l'oléate d'oléyle, de l'huile d'amande douce, de l'huile de tournesol, ou des triglycérides à chaîne moyenne.

Les quatre émulsions préparées avec les différentes huiles sont toutes stables et fluides à 4°C.

### Exemple n° 3

Cet exemple est destiné à illustrer la préparation d'émulsions eau-dans-l'huile stables avec comme tensioactif une huile de ricin hydrogénée polyoxyéthylénée.

Dans 800 ml d'oléate d'oléyle (Cétiol, Henkel) contenant 5 % p/v d'huile de ricin hydrogénée polyoxyéthylénée (7 OE) (Dehymuls HRE7, Henkel), 200 ml de tampon isotonique sont émulsionnés à l'aide d'un émulsionneur à turbine (Silverson).

L'émulsion obtenue est stable à 4°C (pas de rupture après plus d'un an), fluide (60 mPa.s à 25°C).

L'utilisation du dioléate de propylèneglycol (Radia 7204, Oléofina) à la place de l'oléate d'oléyle conduit au même résultat. Avec le dioléate de propylèneglycol par exemple, il est possible d'employer des huiles de ricin polyoxyéthylénées contenant, par mole, 5 résidus d'oxyde d'éthylène (Etocas 5, Croda) à 9 résidus (Acconon CA-9, Karlshamns).

Toutefois, les émulsions préparées dans les mêmes conditions avec des triglycérides (huiles végétales ou bien tricaprylate/tricaprate) en l'absence de cotensioactif ne sont pas stables. De plus, certaines huiles de ricin polyoxyéthylénées ne conduisent pas seules à des émulsions eau-dans-l'huile stables et on préfèrera les employer conformément à l'invention en combinaison avec d'autres tensioactifs classiques ou selon l'invention.

### Exemple n° 4

Cet exemple est destiné à mettre en évidence l'influence de la concentration en polyricinoléate de polyglycérol sur la stabilité des émulsions.

Des émulsions ont été préparées avec 80 % v/v de phase huileuse (oléate d'oléyle) renfermant de 1 à 8 % (p/v) de polyricinoléate de polyglycérol. Après trois mois à 4°C, celles renfermant de 3 à 8 % (p/v) de tensioactif dans la phase huileuse sont plus particulièrement stables.

Le même résultat est obtenu en remplaçant l'oléate d'oléyle par de l'huile d'amande douce.

### Exemple n° 5

Cet exemple est destiné à montrer la possibilité d'associer un polyricinoléate de polyglycérol à un monoglycéride.

Deux phases huileuses contenant l'une 5 % (p/v) de polyricinoléate de polyglycérol (Radiamuls 2253, Oléofina) dans l'huile d'amande douce, l'autre 5 % (p/v) de monooléate de glycérol (Radiasurf 7150, Oléofina) dans l'huile d'amande douce sont mélangées en proportions variables. Puis 40 ml de phase aqueuse sont émulsifiés dans 160 ml de chacune des différentes phases huileuses préparées, a l'aide d'un émulsionneur à turbine (Silverson). Alors que l'émulsion ne contenant que le monooléate de glycérol devient hétérogène, voire se casse en quelques jours, celles contenant au moins 1,6 % final de Radiamuls 2253 sont stables à 4°C et à température ambiante. La formule contenant 2 % final de chacun des 2 tensioactifs a une viscosité de 130 mPa.s à 25°C.

### Exemple n° 6

Cet exemple est destiné à montrer la possibilité d'associer un polyricinoléate de polyglycérol à une huile de ricin polyoxyéthylénée.

Les conditions sont les mêmes que dans l'exemple 5, mais ici l'huile est de l'huile de soja et l'une des deux phases contient, à la place du monooléate de glycérol, de l'huile de ricin hydrogénée polyoxyéthylénée à 7 OE (Dehymuls HRE 7, Henkel). Les émulsions contenant au moins 1,6 % final de Radiamuls 2253 sont stables à 4°C et à température ambiante. La viscosité, pour une émulsion contenant 1,6 % final de Radiamuls 2253, est 120 mPa.s à 25°C.

## Revendications

1. Vaccin ou médicament immunologique formé d'une émulsion injectable eau-dans-l'huile à activité adjuvante de l'immunité, présentant une très bonne tolérance, une grande stabilité, et comportant une phase huileuse avec agent émulsionnant ou tensioactif émulgateur et une phase aqueuse incorporant un antigène vaccinal ou un principe actif immunologique, caractérisée en ce que l'huile de la phase huileuse est une huile métabolisable ou un mélange d'huiles métabolisables et l'agent émulsionnant, ou mélange d'agents émulsionnants, est à base d'ester de l'acide ricinoléique et/ou d'ester de polyglycérol et forme avec l'huile métabolisable et la phase aqueuse, une émulsion stable et à viscosité relativement faible de moins de 400 mPa.s à 25°C.

2. Vaccin ou médicament immunologique selon la revendication 1, caractérisé en ce que l'agent émulsionnant comprend de l'ester d'acide ricinoléique, cet ester étant en tout ou partie de l'huile de ricin polyoxyéthylénée, hydrogénée ou non.

3. Vaccin ou médicament immunologique selon la revendication 1 ou 2, caractérisé en ce que l'agent émulsionnant comprend de l'ester de polyglycérol, cet ester étant en totalité ou en partie des esters de polyglycérol et d'acides gras naturels, tels que l'acide oléique, l'acide stéarique, l'acide ricinoléique, ou bien d'acides gras ramifiés, tels que l'acide isostéarique.

4. Vaccin ou médicament immunologique selon l'une des revendications 1 à 3, caractérisé en ce que l'agent émulsionnant comprend un ester de polyglycérol qui est un ester de polyglycérol et d'acide isostéarique.

5. Vaccin ou médicament immunologique selon l'une des revendications 1 à 4, caractérisé en ce que l'agent émulsionnant comprend de l'huile de ricin polyoxyéthylénée, hydrogénée ou non, et un ester de polyglycérol et d'acide gras naturel, tel que acide oléique, acide stéarique, acide ricinoléique, ou d'acide gras ramifié, tel que acide isostéarique.

6. Vaccin ou médicament immunologique selon l'une des revendications 1 à 5, caractérisé en ce que l'agent émulsionnant comprend de l'huile de ricin polyoxyéthylénée, hydrogénée ou non, et un ester de polyglycérol et d'acide isostéarique.

7. Vaccin ou médicament immunologique selon l'une quelconque des revendications 1 à 6, caractérisé en ce que l'agent émulsionnant comprend de l'ester d'acide ricinoléique, cet ester comprenant du ricinoléate ou polyricinoléate de polyglycérol.

8. Vaccin ou médicament immunologique selon l'une des revendications 1 à 7, caractérisé en ce que l'agent émulsionnant comprend de l'huile de ricin polyoxyéthylénée, hydrogénée ou non, et du ricinoléate ou polyricinoléate de polyglycérol.

9. Vaccin ou médicament immunologique selon l'une des revendications 2, 5, 6 et 8, caractérisé en ce que les huiles de ricin polyoxyéthylénées, hydrogénées ou non, ont un degré d'éthoxylation inférieur à 18 moles d'oxyde d'éthylène par mole.

10. Vaccin ou médicament immunologique selon l'une quelconque des revendications 1 à 9, caractérisé en ce que les agents émulsionnants ou tensioactifs émulgateurs contiennent en totalité ou en partie des esters de polyglycérol, en proportion finale de 0,4 à 20 % p/v, de préférence entre 1 et 10 % et plus particulièrement entre 3 et 5 % p/v.

11. Vaccin ou médicament immunologique selon l'une quelconque des revendications 1 à 10, caractérisé en ce que le polyglycérol possède de 2 à 12 résidus glycérol, et plus particulièrement de 2 à 5 résidus.

12. Vaccin ou médicament immunologique selon l'une quelconque des revendications 1 à 11, caractérisé en ce que l'ester de polyglycérol peut être associé à d'autres tensioactifs, en particulier l'oléate de sorbitan, les ricins polyoxyéthylénés, hydrogénés ou non, et/ou des tensioactifs naturels.

13. Vaccin ou médicament immunologique selon la revendication 12, caractérisé en ce que les tensioactifs naturels sont la lécithine ou bien les glycérides (mono + di), plus particulièrement les glycérides d'acides gras insaturés (oléique et/ou linoléique majoritaires) et d'acides gras à chaîne moyenne (caprylique et/ou caprique).

14. Vaccin ou médicament immunologique selon l'une des revendications 2, 5, 6, 8 et 9, caractérisé en ce que les huiles de ricin sont en proportion finale de 0,5 à 25 % p/v, de préférence entre 2 et 10 % et plus particulièrement entre 4 et 8 %.

15. Vaccin ou médicament immunologique selon l'une quelconque des revendications 2, 5, 6, 8, 9 et 14, caractérisé en ce que les huiles de ricin polyoxyéthylénées, hydrogénées ou non, peuvent être associées entre elles et/ou à d'autres tensioactifs appartenant à des séries chimiques différentes, en particulier les dérivés du sorbitan, les alcools ou les acides gras polyoxyéthylénés, les glycérides, les lécithines.

16. Vaccin ou médicament immunologique selon l'une quelconque des revendications 1 à 15 caractérisé en ce que la phase huileuse métabolisable est constituée d'hydrocarbures naturels.

17. Vaccin ou médicament immunologique selon l'une quelconque des revendications 1 à 15, caractérisé en ce que la phase huileuse métabolisable est constituée d'huile végétale de la série des triacylglycérols.

18. Vaccin ou médicament immunologique selon l'une quelconque des revendications 1 à 15, caractérisé en ce que la phase huileuse métabolisable est constituée d'esters d'acides gras et d'alcools.

19. Vaccin ou médicament immunologique selon la revendication 18, caractérisé en ce que les alcools entrant dans la composition des esters, sont seuls ou en mélange :
- des polyols, tels que le pentaérythritol, le glycérol, le propylèneglycol ;
- des monoalcools aliphatiques à chaîne linéaire, tels que l'isopropanol et les alcools primaires, comme l'éthanol, le butanol, l'octanol-1, le décanol-1, l'alcool oléique ;
- des monoalcools aliphatiques à chaîne ramifiée, tels que l'éthyl-2 hexanol-1 et l'alcool dit isocétylique.

20. Vaccin ou médicament immunologique selon la revendication 18 ou 19, caractérisé en ce que les acides gras majoritaires entrant dans la composition des esters sont des acides naturels de C6 à C24, de préférence l'acide oléique et/ou linoléique, l'acide caprique et/ou caprylique.

21. Vaccin ou médicament immunologique selon la revendication 18 ou 19, caractérisé en ce que les acides gras majoritaires entrant dans la composition des esters sont des diacides tels que l'acide succinique ou l'acide adipique, ou bien des acides gras ramifiés, et plus particulièrement l'acide isostéarique.

22. Vaccin ou médicament immunologique selon l'une quelconque des revendications 1 à 21, caractérisé en ce que la phase aqueuse de l'émulsion est en proportion telle que la viscosité de l'émulsion soit acceptable, pour que ladite émulsion puisse être facilement injectée, de préférence entre 5 et 50 % et plus particulièrement entre 12 et 25 % v/v.

23. Vaccin ou médicament immunologique selon les revendications 1 à 22, caractérisé en ce que l'émulsion contient des substances modulatrices de l'immunité.

24. Vaccin selon l'une quelconque des revendications 1 à 23, caractérisé en ce que la phase aqueuse de l'émulsion contient, seuls ou en association, des antigènes entiers d'origine virale, bactérienne, parasitaire, ou bien des fractions desdits antigènes, ou des protéines obtenues par recombinaison génétique.

25. Médicament immunologique selon l'une quelconque des revendications 1 à 23, caractérisé en ce que la phase aqueuse contient une protéine étrangère, ou une protéine modifiée, ou un haptène conjugué à une molécule porteuse.

26. Vaccin ou médicament immunologique formé d'une émulsion eau-dans-l'huile injectable possédant une activité adjuvante, comprenant une phase aqueuse incorporant un antigène vaccinal ou un principe actif immunologique et une phase huileuse comportant huile et agent émulsionnant, l'huile de la phase huileuse étant substantiellement une huile métabolisable ou un mélange d'huiles métabolisables et l'agent émulsionnant, ou mélange d'agents émulsionnants est à base d'ester de l'acide ricinoléique, en particulier huile de ricin polyoxyéthylénée, éventuellement hydrogénée, et/ou d'ester de polyglycérol, et forme, en mélange avec la phase huileuse, une émulsion stable de viscosité inférieure à 400 mPa.s à 25°C.

## Claims

1. Vaccine or immunological medicament formed from an injectable water-in-oil emulsion possessing immunity adjuvant activity which has very good tolerance and high stability which contains an oily phase with emulsifying agent or emulsifying surface-active agent and an aqueous phase incorporating a vaccinal antigen or an immunological active principle, characterized in that the oil of the oily phase is a metabolizable oil or a mixture of metabolizable oils and the emulsifying agent, or mixture of emulsifying agents, is based on ricinoleic acid ester and/or on polyglyceryl ester and forms, with the metabolizable oil and the aqueous phase, a stable emulsion which has a relatively low viscosity of less than 400 mPa·s at 25°C.

2. Vaccine or immunological medicament according to Claim 1, characterized in that the emulsifying agent comprises ricinoleic acid ester, this ester being, in all or in part, optionally hydrogenated polyoxyethylenated castor oil.

3. Vaccine or immunological medicament according to Claim 1 or 2, characterized in that the emulsifying agent comprises polyglyceryl ester, this ester being, in all or in part, esters of polyglycerol and of natural fatty acids, such as oleic acid, stearic acid or ricinoleic acid, or else of branched fatty acids, such as isostearic acid.

4. Vaccine or immunological medicament according to one of Claims 1 to 3, characterized in that the emulsifying agent comprises a polyglyceryl ester which is an ester of polyglycerol and of isostearic acid.

5. Vaccine or immunological medicament according to one of Claims 1 to 4, characterized in that the emulsifying agent comprises optionally hydrogenated polyoxyethylenated castor oil and an ester of polyglycerol and of natural fatty acid, such as oleic acid, stearic acid or ricinoleic acid, or of branched fatty acid, such as isostearic acid.

6. Vaccine or immunological medicament according to one of Claims 1 to 5, characterized in that the emulsifying agent comprises optionally hydrogenated polyoxyethylenated castor oil and an ester of polyglycerol and of isostearic acid.

7. Vaccine or immunological medicament according to any one of Claims 1 to 6, characterized in that the emulsifying agent comprises ricinoleic acid ester, this ester comprising polyglyceryl polyricinoleate or ricinoleate.

8. Vaccine or immunological medicament according to one of Claims 1 to 7, characterized in that the emulsifying agent comprises optionally hydrogenated polyoxyethylenated castor oil and polyglyceryl polyricinoleate or ricinoleate.

9. Vaccine or immunological medicament according to one of Claims 2, 5, 6 and 8, characterized in that the optionally hydrogenated polyoxyethylenated castor oils have a degree of ethoxylation which is less than 18 mol of ethylene oxide per mole.

10. Vaccine or immunological medicament according to any one of Claims 1 to 9, characterized in that the emulsifying agents or emulsifying surface-active agents contain, in all or in part, polyglyceryl esters in a final proportion of 0.4 to 20 % w/v, preferably between 1 and 10 % and more particularly between 3 and 5 % w/v.

11. Vaccine or immunological medicament according to any one of Claims 1 to 10, characterized in that the polyglycerol has from 2 to 12 glycerol residues and more particularly from 2 to 5 residues.

12. Vaccine or immunological medicament according to any one of Claims 1 to 11, characterized in that the polyglyceryl ester can be combined with other surface-active agents, in particular sorbitan oleate, optionally hydrogenated polyoxyethylenated castor oils and/or natural surface-active agents.

13. Vaccine or immunological medicament according to Claim 12, characterized in that the natural surface-active agents are leicthin or else (mono + di) glycerides, more particularly glycerides of unsaturated fatty acids (mostly oleic and/or linoleic) and of medium-chain fatty acids (caprylic and/or capric).

14. Vaccine or immunological medicament according to one of Claims 2, 5, 6, 8 and 9, characterized in that the castor oils are in a final proportion of 0.5 to 25 % w/v, preferably between 2 and 10 % and more particularly between 4 and 8 %.

15. Vaccine or immunological medicament according to any one of Claims 2, 5, 6, 8, 9 and 14, characterized in that the optionally hydrogenated polyoxyethylenated castor oils can be combined with one another and/or with other surface-active agents belonging to different chemical series, in particular sorbitan derivatives, polyoxyethylenated fatty acids or alcohols, glycerides or lecithins.

16. Vaccine or immunological medicament according to any one of Claims 1 to 15, characterized in that the metabolizable oily phase consists of natural hydrocarbons.

17. Vaccine or immunological medicament according to any one of Claims 1 to 15, characterized in that the metabolizable oily phase consists of vegetable oil of the triacylglycerol series.

18. Vaccine or immunological medicament according to any one of Claims 1 to 15, characterized in that the metabolizable oily phase consists of esters of fatty acids and of alcohols.

19. Vaccine or immunological medicament according to Claim 18, characterized in that the alcohols entering into the composition of the esters are, alone or as a mixture:
- polyols, such as pentaerythritol, glycerol or propylene glycol;
- linear-chain aliphatic monoalcohols, such as isopropanol and primary alcohols, such as ethanol, butanol, 1-octanol, 1-decanol or oleyl alcohol;
- branched-chain aliphatic monoalcohols, such as 2-ethyl-1-hexanol and so-called isocetyl alcohol.

20. Vaccine or immunological medicament according to Claim 18 or 19, characterized in that most of the fatty acids entering into the composition of the esters are natural, C6 to C24, acids, preferably oleic and/or linoleic acid or capric and/or caprylic acid.

21. Vaccine or immunological medicament according to Claim 18 or 19, characterized in that most of the fatty acids entering into the composition of the esters are diacids such as succinic acid or adipic acid or else branched fatty acids and more particularly isostearic acid.

22. Vaccine or immunological medicament according to any one of Claims 1 to 21, characterized in that the aqueous phase of the emulsion is in a proportion such that the viscosity of the emulsion is acceptable, so that the said emulsion can easily be injected, preferably between 5 and 50 % and more particularly between 12 and 25 % v/v.

23. Vaccine or immunological medicament according to Claims 1 to 22, characterized in that the emulsion contains immunity-modulating substances.

24. Vaccine according to any one of Claims 1 to 23, characterized in that the aqueous phase of the emulsion contains, alone or in combination, complete antigens of viral, bacterial or parasitic origin, or else fractions of the said antigens, or proteins obtained by genetic recombination.

25. Immunological medicament according to any one of Claims 1 to 23, characterized in that the aqueous phase contains a foreign protein or a modified protein or a hapten conjugated to a carrier molecule.

26. Vaccine or immunological medicament formed from an injectable water-in-oil emulsion possessing adjuvant activity, comprising an aqueous phase incorporating a vaccinal antigen or an immunological active principle and an oily phase containing oil and emulsifying agent, the oil of the oily phase being substantially a metabolizable oil or a mixture of metabolizable oils and the emulsifying agent, or mixture of emulsifying agents, is based on ricinoleic acid ester, in particular optionally hydrogenated polyoxyethylenated castor oil, and/or on polyglyceryl ester and forms, as a mixture with the oily phase, a stable emulsion with a viscosity of less than 400 mPa·s at 25°C.

## Patentansprüche

1. Impfstoff oder immunologisches Medikament, gebildet aus einer injizierbaren Wasser-in-Öl-Emulsion mit immun-unterstützender Aktivität, und der eine sehr gute Toleranz, und eine große Stabilität aufweist und eine ölige Phase mit einem emulgierenden Mittel oder einem oberflächenaktiven Emulgator und eine wäßrige Phase umfaßt, die ein Impfstoff-Antigen oder einen immunologischen Wirkstoff umfaßt, dadurch charakterisiert, daß das Öl der ölige Phase ein metabolisierbares Öl oder eine Mischung aus metabolisierbaren Ölen ist, und daß das emulgierende Mittel oder die Mischung emulgierender Mittel ein Mittel auf der Basis von Estern von Rizinolsäure und/oder Estern von Polyglycerin ist und mit dem metabolisierbaren Öl und der wäßrige Phase eine stabile Emulsion mit einer relativ geringen Viskosität von mindestens 400 mPa.s bei 25°C bildet.

2. Impfstoff oder immunologisches Medikament nach Anspruch 1, dadurch charakterisiert, daß das emulgierende Mittel einen Ester von Rizinolsäure umfaßt, wobei der Ester ganz oder teilweise aus hydriertem oder nicht-hydriertem polyoxyethyliertem Rizinusöl besteht.

3. Impfstoff oder immunologisches Medikament nach Anspruch 1 oder 2, dadurch charakterisiert, daß das emulgierende Mittel Ester von Polyglycerin umfaßt, wobei der Ester ganz oder teilweise aus Estern von Polyglycerin und natürlichen Fettsäuren wie Ölsäure, Stearinsäure, Rizinolsäure oder verzweigten Fettsäuren wie Isostearinsäure besteht.

4. Impfstoff oder immunologisches Medikament nach einem der Ansprüche 1 bis 3, dadurch charakterisiert, daß das emulgierende Mittel einen Polyglycerinester umfaßt, der ein Ester von Polyglycerin und Isostearinsäure ist.

5. Impfstoff oder immunologisches Medikament nach einem der Ansprüche 1 bis 4, dadurch charakterisiert, daß das emulgierende Mittel hydriertes oder nicht-hydriertes polyoxyethyliertes Rizinusöl und einen Ester aus Polyglycerin und einer natürlichen Fettsäure wie Ölsäure, Stearinsäure, Rizinolsäure oder einer verzweigten Fettsäure wie Isostearinsäure umfaßt.

6. Impfstoff oder immunologisches Medikament nach einem der Ansprüche 1 bis 5, dadurch charakterisiert, daß das emulgierende Mittel hydriertes oder nicht-hydriertes polyoxyethyliertes Rizinusöl und einen Ester aus Polyglycerin und Isostearinsäure umfaßt.

7. Impfstoff oder immunologisches Medikament nach einem der Ansprüche 1 bis 6, dadurch charakterisiert, daß das emulgierende Mittel einen Ester von Rizinolsäure umfaßt, wobei dieser Ester das Rizinoleat oder Polyrizinoleat von Polyglycerin umfaßt.

8. Impfstoff oder immunologisches Medikament nach einem der Ansprüche 1 bis 7, dadurch charakterisiert, daß das emulgierende Mittel hydriertes oder nichthydriertes polyoxyethyliertes Rizinusöl und Rizinoleat oder Polyrizinoleat von Polyglycerin umfaßt.

9. Impfstoff oder immunologisches Medikament nach einem der Ansprüche 2, 5, 6 und 8, dadurch charakterisiert, daß die hydrierten oder nicht-hydrierten polyoxyethylierten Rizinusöle einen Ethoxylierungsgrad von weniger als 18 Mol Ethylenoxid pro Mol aufweisen.

10. Impfstoff oder immunologisches Medikament nach einem der Ansprüche 1 bis 9, dadurch charakterisiert, daß die emulgierenden Mittel oder oberflächenaktiven Emulgatoren ganz oder teilweise Polyglycerinester in einem Endanteil von 0,4 bis 20 Gew.-%, bevorzugt zwischen 1 und 10 Gew.-% und besonders bevorzugt zwischen 3 und 5 Gew.-% enthalten.

11. Impfstoff oder immunologisches Medikament nach einem der Ansprüche 1 bis 10, dadurch charakterisiert, daß das Polyglycerin 2 bis 12 Glycerinreste und insbesondere 2 bis 5 Reste enthält.

12. Impfstoff oder immunologisches Medikament nach einem der Ansprüche 1 bis 11, dadurch charakterisiert, daß der Ester von Polyglycerin mit weiteren oberflächenaktiven Mitteln vorliegen kann, insbesondere Sorbitanoleat, hydrierten oder nicht hydrierten polyoxyethylierten Rizinusölen und/oder natürlichen oberflächenaktiven Mitteln.

13. Impfstoff oder immunologisches Medikament nach Anspruch 12, dadurch charakterisiert, daß die natürlichen oberflächenaktiven Mittel Lecithin oder Glyceride (mono + di), insbesondere Glyceride von ungesättigten Fettsäuren (hauptsächlich Ölsäure und/oder Linolsäure) und mittelkettigen Fettsäuren (Caprylsäure und/oder Caprinsäure) sind.

14. Impfstoff oder immunologisches Medikament nach einem der Ansprüche 2, 5, 6, 8 und 9, dadurch charakterisiert, daß die Rizinusöle in einem Endanteil von 0,5 bis 25 Gew.-%, bevorzugt zwischen 2 und 10 Gew.-% und besonders bevorzugt zwischen 4 und 8 Gew.-% vorliegen.

15. Impfstoff oder immunologisches Medikament nach einem der Ansprüche 2, 5, 6, 8, 9 und 14, dadurch charakterisiert, daß die hydrierten oder nicht-hydrierten polyoxyethylierten Rizinusöle untereinander und/oder mit weiteren oberflächenaktiven Stoffen gemischt sein können, die zu verschiedenen chemischen Reihen gehören, insbesondere Sorbitanderivaten, Alkoholen oder polyoxyethylierten Fettsäuren, Glyceriden, Lecithinen.

16. Impfstoff oder immunologisches Medikament nach einem der Ansprüche 1 bis 15, dadurch charakterisiert, daß die metabolisierbare ölige Phase aus natürlichen Kohlenwasserstoffen besteht.

17. Impfstoff oder immunologisches Medikament nach einem der Ansprüche 1 bis 15, dadurch charakterisiert, daß die metabolisierbare ölige Phase aus einem Pflanzenöl der Reihe der Triacylglycerine besteht.

18. Impfstoff oder immunologisches Medikament nach einem der Ansprüche 1 bis 15, dadurch charakterisiert, daß die metabolisierbare ölige Phase aus Estern von Fettsäuren und Alkoholen besteht.

19. Impfstoff oder immunologisches Medikament nach Anspruch 18, dadurch charakterisiert, daß die Alkohole, die in die Zusammensetzung der Ester eingehen, einzeln oder als Gemisch sind:
- Polyole wie Pentaerythrit, Glycerin, Propylenglykol;
- aliphatische lineare Monoalkohole wie Isopropanol und primäre Alkohole wie Ethanol, Butanol, 1-Octanol, 1-Decanol, Ölalkohol;
- aliphatische verzweigte Monoalkohole wie 2-Ethyl-1-hexanol und Isocetylalkohol.

20. Impfstoff oder immunologisches Medikament nach Anspruch 18 oder 19, dadurch charakterisiert, daß die hauptsächlichen in die Zusammensetzung der Ester eingehenden Fettsäuren natürliche C6 bis C24-Säuren sind, bevorzugt Ölsäure und/oder Linolsäure, Caprinsäure und/oder Caprylsäure.

21. Impfstoff oder immunologisches Medikament nach Anspruch 18 oder 19, dadurch charakterisiert, daß die hauptsächlich in die Zusammensetzung der Ester eingehenden Fettsäuren Disäuren sind wie Bernsteinsäure und Adipinsäure oder verzweigte Fettsäuren, und insbesondere Isostearinsäure.

22. Impfstoff oder immunologisches Medikament nach einem der Ansprüche 1 bis 21, dadurch charakterisiert, daß die wäßrige Phase der Emulsion in einem solchen Anteil vorliegt, daß die Viskosität der Emulsion akzeptabel ist, so daß die Emulsion leicht injiziert werden kann, bevorzugt zwischen 5 und 50 Vol.-% und besonders bevorzugt zwischen 12 und 25 Vol.-%.

23. Impfstoff oder immunologisches Medikament nach den Ansprüchen 1 bis 22, dadurch charakterisiert, daß die Emulsion immunmodulatorische Substanzen enthält.

24. Impfstoff nach einem der Ansprüche 1 bis 23, dadurch charakterisiert, daß die wäßrige Phase der Emulsion einzeln oder als Gemisch vollständige Antigene viralen, bakteriellen, parasitären Ursprungs oder Fraktionen der Antigene oder durch genetische Rekombination erhaltene Proteine enthält.

25. Immunologisches Medikament nach einem der Ansprüche 1 bis 23, dadurch charakterisiert, daß die wäßrige Phase ein Fremdprotein oder ein modifiziertes Protein oder ein an ein Trägermolekül gebundenes Hapten enthält.

26. Impfstoff oder immunologisches Medikament, gebildet aus einer injizierbaren Wasser-in-Öl-Emulsion mit einer verstärkenden Aktivität, umfassend eine wäßrige Phase, die ein Impf-Antigen oder einen immunologischen Wirkstoff enthält und eine ölige Phase, die Öl und ein emulgierendes Mittel umfaßt, wobei das Öl der öligen Phase im wesentlichen ein metabolisierbares Öl oder eine Mischung metabolisierbarer Öle ist, und das emulgierende Mittel oder die Mischung emulgierender Mittel auf von Estern von Rizinolsäure basiert, insbesondere von gegebenenfalls hydriertem polyoxyethyliertem Rizinusöl, und/oder Polyglycerinester, und mit der öligen Phase eine stabile Emulsion mit einer Viskosität von weniger als 400 mPa.s bei 25°C bildet.
